# EUROPEAN PATENT APPLICATION

(11) **EP 3 017 750 A1**
(43) Date of publication of application: **11.05.2016**
(21) Application number: 14306782.5
(22) Date of filing: 06.11.2014
(51) Int. Cl.: A61B 3/16, A61B 5/00

(54) **Passive sensing means for a physiological parameter monitoring system**

(71) Applicant: Ophtimalia, 14460 Colombelles (FR)
(72) Inventor: Moreau, Oliver, 14240 Cahagnes (FR); Pasquette, Franck, 14460 Colombelles (FR); Razavet, Xavier, 14610 Cairon (FR); Mezenge, Luc, 14980 Rots (FR); Cauvet, Philippe, 14000 Caen (FR); Biermans, Peter, 14112 Bieville-Beuville (FR)
(74) Representative: Grünecker Patent- und Rechtsanwälte PartG mbB

(57) **Abstract**

The invention relates to a passive sensing means (100) for a contact lens of a physiological parameter monitoring system, for detecting variations of a physiological parameter, in particular intraocular pressure, the passive sensing means (100) forming a resonant circuit comprising an inductor (101) having spires (1011) on a first main side of a carrier substrate and at least one spire (1012) on a second main side of said carrier substrate, opposite the first main side, and at least one capacitor (121, 122, 123, 124, 125, 126), wherein said at least one capacitor (121, 122, 123, 124, 125, 126) is coplanar. The invention also relates to a corresponding physiological parameter monitoring system.

## Description

### Field of the invention

The present invention relates to the field of physiological parameter monitoring systems, in particular for monitoring variations of intraocular pressure. The invention relates in particular to a passive sensing means for use in a physiological parameter monitoring system and to a corresponding physiological parameter monitoring system.

### Background of the invention

Intraocular pressure is one of the physiological parameters that allows diagnosis and monitoring of eye diseases such as glaucoma. Recently, portable and non-invasive sensing means and methods have been developed in order to measure daily variations of a patient's intraocular pressure, avoiding invasive surgical procedures where sensing means would need to be implanted in a patient's eye. Furthermore, the portability of non-invasive systems has the advantage that patients are no longer required to be immobilized at a hospital or clinic, but that the physiological parameters can now be continuously monitored in daily life situations.

Non-invasive sensing means known in the art usually comprise a sensing device that can be incorporated in a carrier device, such as a contact lens, which will be carried by a patient for monitoring purposes. Furthermore, the non-invasive sensing device can be used in combination with an external monitoring system that can receive and analyze data from the sensing means.

Different types of non-invasive sensing means for contact lenses are known, among which active sensors using miniaturized low power electronics such as microchips, active strain gages and the like, and therefore requiring an energy source. WO 2011/083105 A1 discloses for instance an active sensor comprising concentric strain gages and an associated microprocessor incorporated in a contact lens.

In contrast thereto, purely passive sensors have been developed in order to avoid using an energy source that might cause discomfort to a patient, for instance due to the generation of radiation in close vicinity of or even in direct contact with the patient's eye. A passive sensor is known from EP 2 412 305 A1, disclosing a portable physiological parameter monitoring system comprising a resonant LC circuit incorporated in a soft contact lens, wherein the resonant LC circuit responds to an external magnetic field generated by a complementary portable device, as known from instance from EP 2 439 580 A1, as well as a base station for analyzing the data acquired by the portable device. This type of passive sensor is known to rely on variations of the resonance frequency of the LC circuit incorporated in the contact lens as a function of variations of the intraocular pressure, as the latter should affect the shape of the surface of the eye and, consequently, also of the soft contact lens resting thereon. In turn, deformations of the soft contact lens should modify a capacitance of the resonant circuit.

However, the integration of sensors, passive or active, in contact lenses has been found to be more complex and more expensive than expected, preventing thus far a commercialization of portable intraocular pressure monitoring systems. A recurrent problem is that sensors are usually manufactured flat and subsequently bent to adopt the spherical cap shape of the over-molded lens, which has been found to create deformed areas in the final lens, for instance rippled edges, and sometimes also misalignments between the electrical components of the sensor. Thus, further to not being comfortable for wearing the lens, these deformations prevent a proper flat placement of the lens against the surface of the eye. As a consequence, the necessary sensitivity of the system to deformations of the surface of the eye cannot be reached.

WO 2009/111726 A2 discloses a surface deformation sensor comprising a contact lens formed by an external rigid layer and an internal soft layer bounded together at their edge, with a gap between the rigid and the soft layers. WO 2009/111726 A2 further discloses a resonant LC circuit formed by an inductive coil and a sensing capacitor, wherein the inductive coil and an upper electrode of the capacitor are included in the rigid layer and electrically connected to a lower electrode included in the soft layer. However, the fabrication of this type of surface deformation sensor requires various complex steps of integrating circuit components both in the rigid and in the soft layers, as well as the integration of a mechanism for electrically connecting the two layers.

Thus, an objective of the present invention is to provide an improved passive sensor that can be incorporated in a contact lens of a physiological parameter monitoring system, and a corresponding physiological parameter monitoring system, without the aforementioned problems. In particular, the passive sensor and corresponding monitoring system should also respect common requirements of comfort of wearing and, as much as possible, unimpaired vision of the subject wearing the lens with integrated passive sensor. An objective of the present invention is also to provide a passive sensor that improves the placement of the contact lens against the surface of an eye and responsiveness of the physiological parameter monitoring system to surface deformations.

### General description of the invention

According to an aspect of the invention, the objective is solved with a passive sensing means according to claim 1 or claim 2 for a contact lens of a physiological parameter monitoring system for detecting variations of a physiological parameter. The passive sensing means, which can be for detecting variations of intraocular pressure, forms a resonant circuit comprising an inductor having spires on a first main side of a carrier substrate and at least one spire on a second main side of said carrier substrate, opposite the first main side, and at least one capacitor. According to a first aspect, said at least one capacitor is coplanar. According to a second aspect, the passive sensing means, and in particular the inductor and/or said at least one capacitor, forms first electrodes of at least one sensing capacitor. These two aspects can be taken independently or combined with each other and both solve the aforementioned objective, as explained hereafter.

The use of coplanar conductive elements, in particular at lest one coplanar capacitor, in the passive sensing means provides with a specific and advantageous geometry of the electric field lines generated therein, especially in comparison to passive sensors known in the art having sensing capacitive elements with a substantially face-to-face parallel electrode configuration or with their electrodes arranged on two different layers or planes of the contact lens. Indeed, because of the coplanar circuit elements, the electric field lines can protrude out of the plane of the inventive passive sensing means. Therefore, parasitic capacitances can exist with other surrounding materials having a high relative permittivity when a layer of a low relative permittivity is provided in-between. In particular, the present invention takes advantage of the high relative permittivity of eye tissue and/or of the tear film thereon in order to provide at least one sensing capacitor for detecting variations of the surface of the eye, as will become more obvious with the description of the embodiments.

In fact, the coplanar elements of the inventive passive sensing means, in other words the at least one coplanar capacitor, and also spires of the inductor in advantageous variants, form first electrodes of sensing capacitors using the parasitic capacitances existing with the underlying surface of the eye and/or tear film thereon when the passive sensing means is attached to a contact lens placed on the eye. In other words, instead of having a physically built-in second sensing electrode in the radial direction towards the eye surface, which would -theoretically- vary following the deformations of the surface of the eye but is in practice less efficient than expected, the inventive passive means provides "physically" only for first sensing electrodes, as its configuration and resulting electric field lines enable that the actual surface of the eye and/or the tear film thereon becomes the second sensing electrodes. Thus, an advantage compared to passive sensing means known in the art is that the present invention does not need any physically built-in second electrodes for the sensing capacitors, as the actual surface of the eye and/or the tear film thereon, which are opposite the inductor and/or the at least one capacitor of the passive sensing means, can be the second sensing electrodes. Thus, the invention allows a more direct and efficient monitoring of the deformations of the surface of the eye than intraocular pressure sensors known in the art.

Further advantageous optional features are described in the dependent claims and will also be described hereafter.

Preferably, said at least one capacitor can comprise a first electrode and a second electrode, and said first and second electrodes can be coplanar with the spires of the inductor on the first main side, in particular only on the first main side. An advantage thereof is to facilitate the manufacturing process of the inventive passive sensing means by providing the electrodes of said at least one capacitor on the same main side of the carrier substrate. In addition, a passive sensing means, wherein the coplanar capacitor is coplanar with spires on one main side of the carrier substrate was found particularly advantageous for the structure of the electric field lines that allow taking advantage of parasitic capacitances forming with the surface of the eye and/or the tear film thereon.

Advantageously, the first main side of the carrier substrate can then be the side of the passive sensing means configured for facing the surface of the eye when the passive sensing means is attached to a contact lens. This configuration is preferred to having the second main side facing the eye, but as long as the thickness of the carrier substrate, and therefore the distance between the spires of the inductor on the first main side and the at least one spire on the second main side, is sufficiently small compared to the distance between the first main side and the surface of the eye, the main varying factor in the resonance frequency of the passive sensing means can remain the parasitic capacitance between coplanar elements of the first main side and the surface of the eye and/or the tear film thereon.

Preferably, said first electrode can be electrically connected to an inner circumference of the spires on the first main side and said second electrode can be electrically connected, in particular by means of an electrically conductive via, to said at least one spire on the second main side. Therefore, while the second main side of the carrier substrate is mainly used for the at least one other spire of the inductor, it can also be used advantageously for providing electrical connections with the conductive elements provided on the first main side of the carrier substrate. For instance, the first electrodes of said at least one capacitor can be integral with the innermost spire of the inductor on the first main side. Furthermore, electrical connections between the conductive elements on the first main side and those on the second main side can be integral with the at least one spire on the second main side of the carrier substrate and comprise electrically conductive vias passing through the substrate and joining the conductive elements on the two main sides thereof.

Preferably, said at least one capacitor and/or said first electrode and second electrode can be interdigitated. Following preferred variants, a coplanar interdigitated capacitor can have its electrodes interdigitated radially and/or circumferentially. In particular, the two electrodes of a coplanar capacitor could be interdigitated with one another radially, or a first electrode could itself be interdigitated while being circumferentially coplanar with the second electrode. Interdigitated capacitors, which can also be coplanar, or more in general capacitors with interdigitated electrodes, were found advantageous to improve the sensitivity of the passive sensing means, while also providing an advantageous geometry of the electric field lines.

Preferably, said at least one capacitor can be provided at an inner circumference of the inductor, in particular of the spires on the first main side, more in particular towards a central area of the passive sensing means. Thus, in advantageous embodiments, the inductor can provide for first electrodes of sensing capacitors on circumferential areas of the surface of the eye, which can include peripheral areas of the cornea, and the at least one physical capacitors can provide for first electrodes of sensing capacitors covering a surface within an inner circumference of the inductor, preferably over the cornea.

In a further variant, said at least one capacitor can be larger towards the inner circumference of the inductor than towards said central area. In preferred embodiments, a trapezoidal-like geometry of said at least one capacitor was found advantageous, as it can be easily bent to follow the concave cap geometry of a contact lens. The latter geometry was found advantageous in particular in combination with a circular ring-shaped inductor. In another variant, said at least one capacitor can be partially arc-shaped following said inner circumference. Thus, the geometry of coplanar capacitors can advantageously be adapted to that of the inductor, in particular to that of the spires on the first main side, in order to increase the coverage of the surface of an eye, while still leaving at least a central zone free to allow for a sufficiently unimpaired vision. In particular, it is advantageous to adapt the geometry so that, once the passive sensing means is bent and attached to a contact lens, the initially coplanar elements cover as much of the underlying eye surface as possible while not impairing the vision.

Preferably, the inductor can comprise fewer spires on the second main side than on the first main side of the carrier substrate. While having similar amounts of spires was thought to be advantageous in passive sensors known in the art, with the inventive passive sensing means, it is more advantageous to have as many spires as possible only on the first main side, in other words on the side where they are coplanar with said at least one capacitor. At least one spire, and sometimes a few more spires, are then provided on the second main side of the carrier substrate in order to increase the amplitude of the signal received by a complementary external antenna device -which can be the device generating the external magnetic field- while keeping the size, in particular the diameter, of the passive sensing means sufficiently small to be incorporated in a contact lens for use on a human eye.

Advantageously, said at least one spire on the second main side of the carrier substrate can be provided only at an outer circumference. In order to increase the signal amplitude, it is more advantageous to provide the at least one spire on the second main side only towards the outer circumference of the passive sensing means. Thus, several variants are possible, all compatible with each other. In one variant, in the thickness direction of the carrier substrate, the at least one spire of the second main side can be superimposed with at least one spire of the first main side. For instance, the outermost spires on either side of the carrier substrate can be superimposed. In another variant, it is also possible that the at least one spire of the second main side can be provided on a larger circumference than any of the spires of the first main side.

In some embodiments, the inductor can be ring-shaped and circular. This variant can be advantageous to increase the amplitude of the signal at the antenna of a complementary portable device generating the external magnetic field.

In alternative embodiments, the inductor can be a flat spiral inductor comprising a plurality of, preferably three, concave arc-shaped segments with respect to a substantially central point of said passive sensing means, and wherein for at least one, preferably all, of the plurality of concave arc-shaped segments, the radius of curvature of said at least one segment at a point thereof is greater than the distance between said point and said substantially central point. Here, by the expression "arc-shaped", it should be understood that each arc-shaped inductor segment has, respectively, a curved geometry that follows essentially the shape of an arc of an ellipse, in particular an arc of a circle. Furthermore, while each arc-shaped segment can preferably be a continuous arc-shaped segment, a plurality of shorter back-to-back linear segments could also realize one longer segment having a globally arc-shaped geometry, which would also allow carrying out the invention. Also, by the expression "concave with respect to a substantially central point" and the like, it should be understood that the arc-shaped segments are all concave with respect to a same reference point of the passive sensing means, which can be about the geometrical center thereof, but which is not the center of any of the arc-shaped segments. Thus, according to the invention, the concave arc-shaped segments are not on a circle centered on this reference substantially central point.

Thus, in a preferred embodiment, the inventive passive sensing means can have an inductive element with a structure comprising a plurality, preferably three, flap or ear-like segments that can be better adapted to the concave cap shape of a contact lens than inductors of known sensors because they allow controlling the areas of the passive sensing means that will be bent, folded and/or plastically deformed during the incorporation or attachment to a lens. Given the dimensions of contact lenses and therefore the requirements on the dimension of passive sensing means, three concave arc-shaped inductor segments can provide a better compromise in terms of sensitivity and surface coverage, as well as in terms of flexibility for the incorporation of the sensor in a contact lens than more or less such segments. However, two, four or more concave arc-shaped segments with large radii should not be ruled out in variants of preferred embodiments. Furthermore, the curvature radii of the concave arc-shaped segments of the inductor can advantageously be chosen such that, once the passive sensing means is deformed for its incorporation in a contact lens, they will essentially describe segments of a same predetermined circle of the contact lens, which allows easier placement in the contact lens.

In a variant of a preferred embodiment, the inductor can further comprise convex arc-shaped segments arranged between the concave arc-shaped segments. Here, the expression "convex arc-shaped segments" should be understood in a manner similar to "concave" as explained above. Thus, the convex arc-shaped segments are convex with respect to a substantially central point of the passive sensing means, as explained above. In this way, the areas where the passive sensing means can be bent during an incorporation or attachment process to a contact lens can be controlled.

In a further variant, the inductor can further comprises straight segments joining said convex arc-shaped segments to said concave arc-shaped segments, and the junctions between said straight segments and the concave arc-shaped segments can preferably be rounded. The length of the joining straight inductor segments can be used to better control the amount of material between the concave arc-shaped segments. Rounded junctions between successive inductor segments provide smoother shapes than rough pointy edges and are thus easier to attach to the concave cap shape of a contact lens. Here, attention should be brought to the fact that, while in this variant the rounded junctions could thus be concave-shaped, they are however not concave "with respect to the center point", unlike the "concave arc-shaped segments" as explained above.

Preferably, the inductor can comprise 5 to 20 spires, in particular 8 to 15 spires, more in particular 10 to 13 spires, on the first main side and one or more, in particular up to 5, spires on the second main side of the carrier substrate. Also, in preferred embodiments of this variant, the width of the spires and/or the distance between spires on either main side of the carrier substrate can be in a range from about 30 µm to about 100 µm, preferably about 40 µm to about 80 µm. Thus, the invention allows combinations of number of spires and dimensions that can advantageously allow a subject wearing a contact lens with the inventive passive sensing means to keep a clear vision. In particular, it is possible but not necessary that the width of the spires and the distance between successive spires are the same. Advantageously, the width of the inductor can be about 2 mm or less, preferably about 1.5 mm or less. The width of the inductor can in fact be greater than this value, but it is more advantageous that it is kept lower in order to keep the subject's vision clear.

In an advantageous variant of a preferred embodiment, for at least one, preferably all, of the plurality of inductor concave arc-shaped segments, at least one capacitor can be provided at an inner circumference of said inductor concave arc-shaped segment towards a central area of said passive sensing means. This arrangement was found advantageous for bending the passive sensing means in view of its attachment to a contact lens. While it is possible that the passive sensing means works with only one capacitor, it is more advantageous in terms of sensitivity to include more than one capacitor. In a preferred variant, it is therefore possible to provide at least one capacitor at an inner side of each inductor concave arc-shaped segment. A configuration with two capacitors for each of the inductor concave arc-shaped segment was found even more advantageous in terms of sensitivity and surface coverage, while providing for sufficient visibility for a subject wearing a contact lens with the inventive passive sensing means.

Preferably, the passive sensing means can further comprise a central area free of inductor and/or capacitor material. Thus, a subject can keep a substantially clear vision while wearing a contact lens with the inventive passive sensing means. The central area can be an area corresponding roughly to the average dimensions of the human pupil.

Advantageously, the passive sensing means can further comprise a layer of a coating material over said inductor and said at least one capacitor and/or over the carrier substrate layer. The coating layer can be advantageous for protecting the circuit components, for instance from corrosion due to prolonged exposure to tears. Furthermore, the carrier substrate and/or the coating can preferably be removed following preferred contours of the passive sensing means. The problem of incorporating or attaching the passive sensing means to a contact lens is somewhat similar to wrapping a 3D surface with a 2D sheet. It is therefore advantageous to remove areas of carrier substrate that would create unnecessary material and therefore form ripples when deforming the passive sensing means to give it a curved shaped prior to its incorporation or attachment to a contact lens. It is in fact preferable to remove as much carrier substrate as possible in order to make the passive sensing means as flexible as possible prior to its incorporation in a contact lens, while still leaving sufficient carrier substrate material in fragile areas, which could be subject to possible tears when the passive sensing means is bent.

According to another aspect of the invention, the objective is also solved with a physiological parameter monitoring system according to claim 16. The Physiological parameter monitoring system, which can be for detecting variations of intraocular pressure, comprises a first lens element with an inner surface and an outer surface opposite the inner surface, wherein at least the outer surface is adapted for contacting an ocular tissue, in particular eyelid tissue, and wherein, preferably, the inner surface is adapted for contacting at least the cornea and/or a tear film thereon, preferably the cornea and sclera and/or a tear film thereon. Preferably, the first contact lens element provides an intermediate space between its inner surface and the surface of an eye when the peripheral area is contacting the sclera. The physiological parameter monitoring system further comprises a passive sensing means according to the previous aspect or any of its variants.

Thus, the physiological parameter monitoring system comprises the advantages of the passive sensing means according to the first aspect of the invention. In particular, the passive sensing means according to the first aspect of the invention provides for first electrodes of at least one sensing capacitor, and the intermediate space can be an intermediate dielectric such that the surface of the eye or the tear film thereon forms second electrodes of the sensing capacitors.

Preferably, the passive sensing means can be provided at the inner surface of the first contact lens element, preferably with the second main side of the carrier substrate towards said inner surface. Whether attached to the internal optical surface of a rigid contact lens, in particular a rigid scleral contact lens, or accommodated in a recess therein, the present invention does not require complex steps of incorporation of the passive sensing means within the lens material, or of over-molding contact lens layers on the passive sensing means.

In preferred variants of advantageous embodiments, the physiological parameter monitoring system can further comprise a second lens element, preferably of a flexible material, in particular a flexible polymer material, more in particular a hydrophilic flexible polymer material, having an inner surface and an outer surface opposite the inner surface, wherein at least the inner surface can be adapted for contacting an ocular tissue, in particular at least the cornea and/or a tear film thereon, and wherein the first lens element and the second lens element can be attached to one another at a peripheral attachment area, thereby enclosing an intermediate space. Thus, the inventive system can take better advantage of a multilayered contact lens than surface deformation sensors known in the art. Indeed, the inventive passive sensing means is incorporated or attached only to the rigid part of a multilayered contact lens, thereby advantageously avoiding to have to incorporate or attach any circuit element to the soft layer of the lens, which improves the flat placement of the soft layer against the surface of an eye in comparison to systems known in the art, as the soft layer no longer integrates stiffening elements. Thus, the formation of ripples is also avoided in the soft layer. In addition, the integration of a mechanism for electrically connecting circuit elements in the soft layer to circuit elements in the rigid layer is also avoided with the inventive passive sensing means.

Thus, depending on the variant, it is even possible to use only a rigid contact lens, in particular a rigid scleral contact lens, without any soft contact lens layer, as the inventive passive sensing means with circuit elements arranged in a coplanar manner can even allow a detection of surface deformations without using a soft contact lens layer as sensing layer. In other variants, also depending on the resonance frequency, using a multilayered contact lens as described above can be more advantageous. In all variants, the contact lens(es) can be corrective or not.

In a variant, when the physiological parameter monitoring system comprises a multilayered contact lens, the intermediate space can be filled with a dielectric material. It is then also preferable that the dielectric material be compressible such that, when the second lens element is flexible, deformations of the underlying surface can still be detected. In fact, following preferred variants, the intermediate space could be fully filled with a compressible dielectric material or partially filled with a mixture of compressible and incompressible dielectric materials, such that the deformations of the underlying surface can be detected. Although multilayered contact lenses known in the art usually enclose an intermediate space filled with air, it is always possible to fill said space with another dielectric material, preferably having also a low relative permittivity. In particular, the dielectric material can have a relative permittivity value, εᵣ, of less than the relative permittivity of a tear film and/or an ocular tissue at ambient temperature, preferably less than about 10 times the relative permittivity of a tear film and/or ocular tissue at ambient temperature, more preferably a relative permittivity value, εᵣ, between about 1 and about 5. Advantageously, decreasing the relative permittivity can increase the sensitivity.

Advantageously, in a variant of a preferred embodiment, the second contact lens element can be a soft contact lens, in particular extending at least over the cornea. Thus, it is even possible to use directly a corrective or non-corrective soft contact lens and attach the same to the first contact lens element, which avoids further complex steps of manufacturing dedicated soft layers. An advantage thereof is that using directly existing soft contact lenses can avoid also completely the problem of ripple formation and flat placement against at least the cornea. This variant was found to be particularly adapted for monitoring deformations of the surface of the eye, and therefore also variations of the intraocular pressure.

In preferred variants of advantageous embodiments, the second contact lens element can extend over the cornea and part of the sclera leaving a non-contact area at the limbus. Most soft so-called corneal contact lenses are in fact also partially scleral and can therefore also be used in this variant. Leaving a non-contact area at the limbus of the eye can provide for a small depression allowing the second lens element, in this variant for instance a soft layer, in particular a soft contact lens, to stick flat against the surface of at least the cornea with help of the tear film.

### List of Figures

The invention will be described in more detail in the following, based on advantageous embodiments described in combination with the following figures:
- Figure 1: schematically illustrates an exemplary embodiment of a passive sensor according to an aspect of the invention;
- Figure 2: schematically illustrates an exemplary embodiment of a physiological parameter monitoring system according to another aspect of the invention;
- Figure 3: schematically illustrates a variant of the physiological parameter monitoring system illustrated in Figure 2, in another exemplary embodiment;
- Figure 4A: schematically illustrates a further variant of the physiological parameter monitoring system illustrated in Figure 2, in another exemplary embodiment;
- Figure 4B: schematically illustrates a variant of the physiological parameter monitoring system illustrated in Figure 4A, in another exemplary embodiment;
- Figure 5: schematically illustrates a variant of the passive sensor illustrated in Figure 1, in a further exemplary embodiment;
- Figure 6: schematically illustrates another exemplary embodiment of a variant of a passive sensor according to an aspect of the invention; and
- Figure 7: schematically illustrates a variant of the passive sensor illustrated in Figure 6, in another exemplary embodiment.

### Description of embodiments

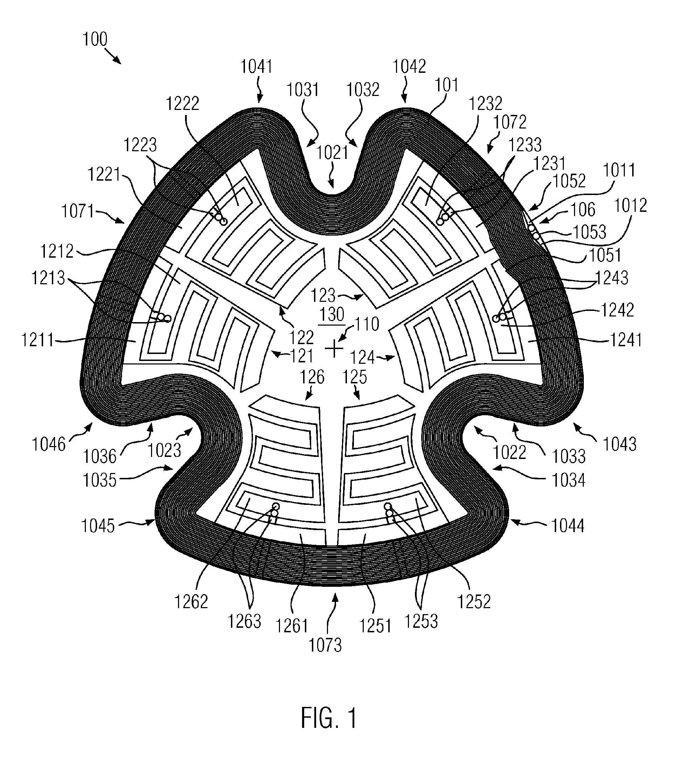

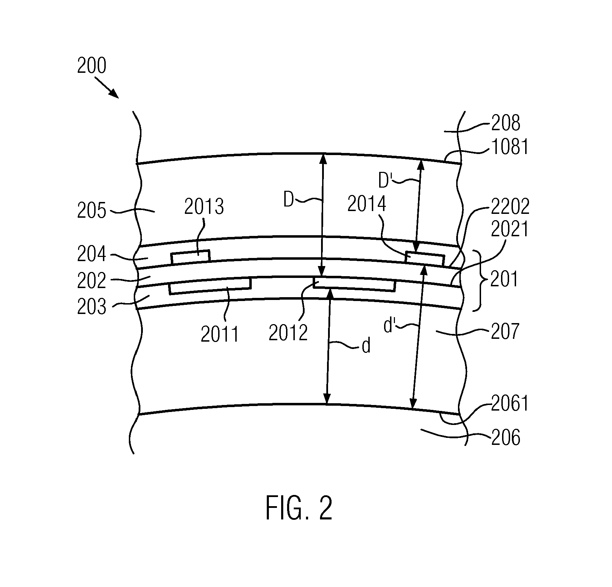

Figure 1 illustrates a passive sensing means 100 for a contact lens, which can be used in a physiological parameter monitoring system, for instance those detailed in the embodiments with reference to Figures 2 to 4B, in an exemplary embodiment of the first aspect of the invention. In this embodiment, the passive sensing means 100 is a resonant circuit for use in a contact lens for detecting variations of a physiological parameter when the contact lens is being worn. In particular, the passive sensing means 100 can be used in a contact lens for monitoring variations of the intraocular pressure, for instance for patients suffering from glaucoma.

As can be seen in Figure 1, the passive sensor 100 comprises an inductive element, here inductor 101, and at least one capacitive element, here the plurality of capacitors 121, 122, 123, 124, 125 and 126, which are all provided as coplanar capacitors. Prior to subsequent steps of incorporation in a contact lens, the passive sensor 100 is sufficiently thin to be considered substantially flat such that it forms essentially only one sensing layer, also when it is deformed, in particular bent, and attached to a contact lens of a physiological parameter monitoring system.

Furthermore, in the embodiment illustrated in Figure 1, the inductor 101 comprises a plurality of spires 1011 on a first main side of a carrier substrate and at least one other spire 1012 on a second main side of the carrier substrate, which is opposite the first main side. In addition, the passive sensor 100 can optionally be provided with a coating layer, for instance on either main side of the carrier substrate or even on both sides thereof, in particular also over the inductor 101 and/or the capacitors 121, 122, 123, 124, 125, 126. For the sake of simplicity, the carrier substrate and optional coating layer(s) are, however, not illustrated in Figure 1. In the embodiment illustrated in Figure 1, on the second main side of the carrier substrate, the inductor 101 forms one spire 1012, which starts from a terminal 1052 on the outermost circumference, and which is then connected at its end to the beginning of the outermost spire among the spires 1011 of the first main side of the carrier substrate by means of one or more electrically conductive vias 1053 passing through the carrier substrate. The inductor 101 then continues to spiral on the first main side of the carrier substrate, in the same spiral direction as on the second main side, with its outermost spire essentially overlapping the spire 1012 of the second main side, towards the central area 130. The spires 1011 of the first main side then end with another terminal 1051 on their innermost circumference. The area 106 between the two terminals 1051, 1052 can present small deflections between successive spires, as illustrated in Figure 1, in particular between successive spires 1011 on the first main side.

In the embodiment illustrated in Figure 1, only one spire 1012 is provided on the second main side of the carrier substrate. However, in other embodiments, at least one outermost spire 1012 on the second main side of the carrier substrate could be provided on a larger circumference than that of any of the spires 1011 on the first main side. In such variants, it would then be also possible to have more than one spire 1012 on the second main side, for instance up to five, as long as there are more spires 1011 on the first main side than spires 1012 on the second main side. On the first main side, the inductor 101 can comprise for instance about 5 to 20 spires 1011, preferably 8 to 15 spires 1011, more preferably 10 to 13 spires 1011. Thus, in the embodiment illustrated in Figure 1, the inductor 101 comprises for instance 12 spires 1011 on the first main side, and only one spire 1012 on the second main side.

As further illustrated in Figure 1, in this embodiment, the inductor 101 can comprise a plurality of segments 1071, 1072, 1073 that are arc-shaped and concave with respect to a reference point, here the substantially central point 110 of the passive sensor 100, wherein this central point 110 does not need to be the geometric center of the sensor but can be close to it. As further illustrated in Figure 1, these segments 1071, 1072, 1073 are in fact not centered on said substantially central point 110. Indeed, at least one segment 1071, 1072, 1073, and preferably all three segments 1071, 1072, 1073, has a curvature radius at a point thereof that is greater than the distance of said point to the substantially central point 110. Thus, following a preferred variant, the centers of the concave arc-shaped inductor segments 1071, 1072, 1073 can in fact be even outside the perimeter of the inductor 101. The inductor 101 then has the advantage that the flap-like or ear-like structure of the three segments 1071, 1072, 1073 will be easier to attach or to incorporate to the concave cap shape of a contact lens. In fact, it will be even possible to bend the sensor 100 such that the segments 1071, 1072, 1073 can substantially align on the same circle in the contact lens.

As also illustrated in Figure 1, in order to further facilitate the attachment or incorporation process of the passive sensor 100 in a contact lens, in particular to better control the areas that will bend during this process, the inductor 101 of the passive sensor 100 can further comprise inwards orientated, in other words convex with respect to the substantially central point 110, arc-shaped segments 1021, 1022, 1023 joining the concave segments 1071, 1072, 1073 to one another. Depending on the desired size of the passive sensor 100, Figure 1 also illustrates that it is possible to join the concave segments 1071, 1072, 1073 to the convex segments 1021, 1022, 1023 via straight inductor segments 1031, 1032, 1033, 1034, 1035, 1036. Thus, the depth of the inwards pointing ear-like segments 1021, 1022, 1023 can be adjusted, thereby controlling the areas that will be bent during the attachment or incorporation process in a contact lens. Figure 1 also illustrates an advantageous variant in which the junctions 1041, 1042, 1043, 1044, 1045, 1046 between the straight segments 1031, 1032, 1033, 1034, 1035, 1036 and the concave segments 1071, 1072, 1073 are rounded in order to provide a smoother shape.

Furthermore, following another preferred variant, since it is desirable that the total width of the inductor 101 in a radial direction, that is for instance with respect to central point 110, is kept below about 2.0 mm, for instance at about 1.5 mm or even below, in the embodiment illustrated in Figure 1, the width of a spire 1011, 1012 can be about 50 µm, while the distance between successive spires 1011 on the first main side can also be about 50 µm. However, in other embodiments, the width of the spires 1011, 1012 and/or the distance between successive spires 1011 or 1012 could be chosen in a range from about 30 µm to about 100 µm, preferably between about 40 µm and about 80 µm and don't need to be the same. Even if it is preferable that the spires 1011, 1012 on the first and second main sides of the carrier substrate have similar dimensions, in some embodiments the width and distance between spires 1011, 1012 on the first and second main sides could be different.

As further illustrated in Figure 1, in contrast to capacitors with a face-to-face parallel electrode configuration, the capacitors 121, 122, 123, 124, 125, 126 are coplanar capacitors, meaning that their respective electrodes 1211 and 1212, 1221 and 1222, 1231 and 1232, 1241 and 1242, 1251 and 1252, and 1261 and 1262, are coplanar to one another, at least before bending or deforming the sensor 100 for its attachment to a contact lens. Following a preferred variant, in this embodiment the coplanar capacitors 121, 122, 123, 124, 125, 126 are also coplanar with the spiral inductor 101 on a main side of the carrier substrate. In particular, the electrodes 1211, 1212, 1221, 1222, 1231, 1232, 1241, 1242, 1251, 1252, 1261, 1262 are coplanar only with the spires 1011 of the inductor 101 on the first main side of the carrier substrate (not illustrated for clarity purposes). Thus, electric field lines between two respective coplanar electrodes 1211 and 1212, 1221 and 1222, 1231 and 1232, 1241 and 1242, 1251 and 1252, 1261 and 1262 can also form arcs protruding out of the plane, and so can electric field lines of the coplanar spires 1011.

Furthermore, following an advantageous variant, the capacitors 121, 122, 123, 124, 125, 126 can also be interdigitated capacitors, as also illustrated in Figure 1. Thus, a given capacitor can comprise two essentially E-shaped electrodes facing each other such that their branches are interdigitated with one another. For instance, in Figure 1, capacitor 121 comprises two essentially E-shaped coplanar and interdigitated electrodes 1211 and 1212. Similarly, the other capacitors 122, 123, 124, 125 and 126 are also provided in this manner.

Also following an advantageous variant, at least one capacitor is provided for each of the concave arc-shaped inductor segments 1071, 1072, 1073, at their inner periphery towards the central point 110. In the embodiment illustrated with reference to Figure 1, following a preferred variant, two capacitors are provided for each concave arc-shaped inductor segments 1071, 1072, 1073. For instance, capacitors 121 and 122 are provided in segment 1071, while capacitors 123 and 124 are provided in segment 1072, and capacitors 125 and 126 are provided in segment 1073. Following a preferred variant, first electrodes of a given capacitor 121, 122, 123, 124, 125, 126, here electrodes 1211 and 1221, 1231 and 1241, and 1251 and 1261, can be electrically connected to an inner side -or inner circumference- of the innermost spire of the spires 1011 of the inductor 101, here to the innermost spire of segments 1071, 1072 and 1073, respectively, on the first main side of the carrier substrate, which is also the spire comprising the terminal 1051. In turn, second electrodes, here electrodes 1212 and 1222, 1232 and 1242, and 1252 and 1262, can be connected to an outer side -or outer circumference- of the inductor 101, here to the outermost spire 1012 of segments 1071, 1072 and 1073, respectively, on the second main side of the carrier substrate, which comprises the other terminal 1052 of the inductor 101. While the first electrodes 1211, 1221, 1231, 1241, 1251, 1261 can be provided substantially as integral extensions of the innermost spire of the plurality of spires 1011 on the first main side of inductor 101 towards the central point 110, the second electrodes 1212, 1222, 1232, 1242, 1252, 1262 can be connected to the spire 1012 of the inductor 101 on the second main side by means of respective electrically conductive vias 1213, 1223, 1233, 1243, 1253, 1263 passing through the carrier substrate, and if necessary even through the second electrodes 1212, 1222, 1232, 1242, 1252, 1262 themselves, as illustrated in Figure 1. As also illustrated in Figure 1, the vias 1213, 1223, 1233, 1243, 1253, 1263 can comprise, respectively, a conductive bridge integral with the spire 1012.

In the embodiment illustrated with reference to Figure 1, the capacitors 121, 122, 123, 124, 125, 126 can be larger towards the innermost spire of the inductor 101 than towards the central point 110, with the larger base facing outwards from the central point 110 and the smaller base facing towards the central point 110 or the central area 130. Furthermore, the capacitors 121, 122, 123, 124, 125, 126 can also be arched so as to broaden again towards the central area 130 -which can be free of material-, as further illustrated in Figure 1. Thus, the capacitors 121, 122, 123, 124, 125, 126 can be partially arc-shaped, in particular following the geometry of the convex arc-shaped segments 1021, 1022, 1023, with the advantage of covering additional surface of the eye than in variants with more straight trapezoidal geometries. This shape can be advantageous for a subsequent bending of the passive sensor 100 in view of its incorporation in a contact lens. This shape is, however, not limitative and other shapes could be used if they facilitate the attachment of the passive sensor 100 to a contact lens or the coverage of the surface of the eye.

It is also preferable to remove unnecessary material from the passive sensor 100 in order to facilitate its incorporation in a contact lens. Thus, it is advantageous to remove at least partially any unnecessary parts of the carrier substrate (not illustrated for clarity purposes), preferably following the inner and outer contours of the passive sensor 100, leaving however sufficient carrier substrate material in areas where bending the passive sensor 100 could damage the inductor 101 and/or any of the capacitors 121, 122, 123, 124, 125, 126. It is also preferable to leave a central area 130 surrounding the substantially central point 110 free of any material, for instance corresponding to the position of the pupil, such that the vision remains essentially unimpaired and the flexibility of the passive sensor 100 is improved.

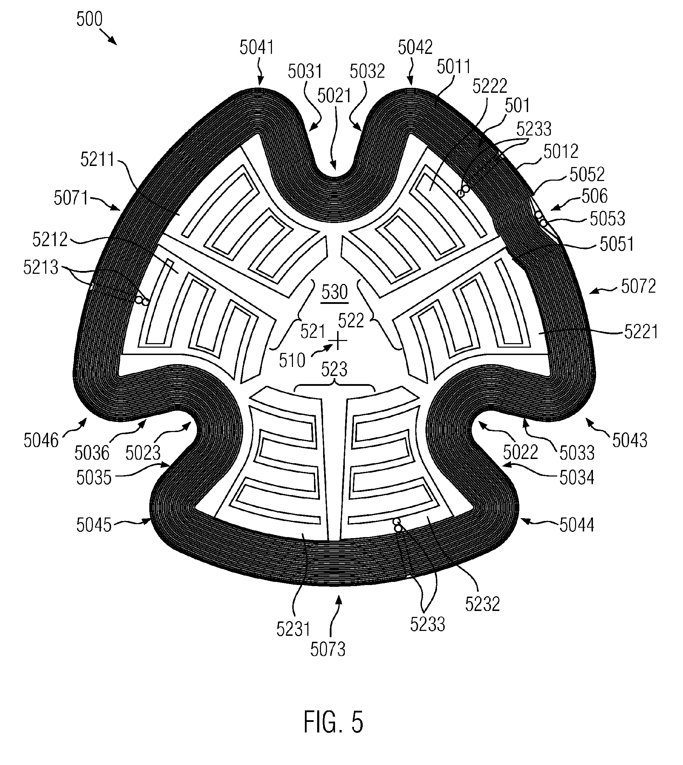

Figure 2 schematically illustrates a detail, in a cross-section, of an exemplary embodiment of a physiological parameter monitoring system 200 according to an aspect of the present invention, in particular using a passive sensing means 201 forming a resonant circuit, for detecting variations of a physiological parameter related in particular to deformations of the surface 2061 of a layer 206 of a high relative permittivity material. In a preferred variant of this embodiment, the passive sensing means 201 can be the passive sensor 100 of the embodiment illustrated with reference to Figure 1. However, variants of the passive sensor 100 or other substantially coplanar passive sensors forming a resonant circuit could be used instead. In particular any of the passive sensors 500, 600, 700of the embodiments illustrated with reference to Figures 5 to 7 could be used in variants of this embodiment.

In the exemplary embodiment illustrated in Figure 2, the passive sensing means 201 comprises a plurality of coplanar conductive elements spread over a first main side 2021 of a carrier substrate 202, which can be inductive and/or capacitive elements, forming a resonant circuit with a given resonance frequency chosen in a range of frequencies preferably adapted for a medical use. For clarity purposes, only two coplanar such elements 2011, 2012 are illustrated in Figure 2. Other conductive elements 2013, 2014 can also be coplanar on a second main side 2022 of the carrier substrate 202, opposite the first main side 2021, but these elements 2013, 2014 are only provided at the outermost circumference of the passive sensing means 201 and not towards the central area thereof. Furthermore, the first main side 2021, having many more conductive coplanar elements spread over its surface, is the side facing the surface 2061 to be monitored. In a preferred variant, when the passive sensing means 201 is the passive sensor 100 of the embodiment illustrated in Figure 1, or any of the passive sensors 500, 600, 700of the embodiments illustrated in Figures 5 to 7, the two coplanar conductive elements 2011, 2012 can correspond to two successive conductive elements on the first main side of the carrier substrate, in a cross-section, for instance two successive spires of the plurality of spires 1011 of the spiral inductor 101 or two successive branches of either of the interdigitated capacitors 121, 122, 123, 124, 125, 126. Similarly, the elements 2013, 2014 can be the spire 1012 on the second main side of the carrier substrate in the embodiment illustrated in Figure 1. Like the embodiments illustrated in Figure 1 and in Figures 5 to 7, the passive sensing means 201 of the exemplary embodiment illustrated in Figure 2 can be provided with an optional layer 203, 204 of a protective coating material being provided on or over the front side 2021 and/or the conductive elements 2011, 2012, as well as on or over the backside 2022 and/or the conductive elements 2013, 2014, respectively. Furthermore, the passive sensing means 201 can be attached to a carrier element, illustrated as the layer 205, of the physiological parameter monitoring system 200 at the backside 2022 of the layer 202 of carrier substrate material. Thus, the optional layer 204 can also be or comprise an adhesive material.

As further illustrated in Figure 2, the passive sensing means 201 attached to the carrier element 205 will be used to determine variations of a physiological parameter related to deformations of the surface 2061 of the first high relative permittivity layer 206. Thus, in the vicinity of the resonance frequency of the passive sensing means 201, the relative permittivity of the carrier 205 and of the layers of substrate 202, coating 203, and coating and/or adhesive 204, are preferably chosen all very low in comparison to the relative permittivity of the first layer 206, for instance preferably at least ten times lower.

The use of coplanar conductive, inductive and/or capacitive, elements 2011, 2012 on the main side 2021 provides with a different electric field lines geometry than capacitors with a face-to-face parallel electrode configuration, such that instead of having essentially straight electric field lines between two opposite parallel electrodes, the electric field lines in the coplanar configuration illustrated in Figure 2 can also protrude out of the plane of the coplanar conductive elements 2011, 2012, for instance following arcs. It is known that parasitic capacitances can exist between the conductive elements 2011, 2012, as well as between the same and any other high relative permittivity elements in vicinity thereof, which can affect the resonance frequency of the passive sensing means 201. In the embodiment illustrated in Figure 2, an intermediate layer 207 is provided between the passive sensing means 201 and the layer 206 of high relative permittivity, wherein the relative permittivity of said intermediate layer 207 is also very low, preferably at least ten times lower, compared to that of the material of layer 206. Thus, since electric field lines can protrude out of the plane of the coplanar elements 2011, 2012, parasitic capacitances can also be formed between each of the coplanar conductive elements 2011, 2012 of the passive sensing means 201 and opposite areas of the surface 2061 of the high relative permittivity layer 206, thereby forming a plurality of sensing capacitors having substantially a parallel electrode configuration, wherein one electrode is one of the conductive elements 2011, 2012 and the other electrode is the opposite area of the surface 2061. In other words, the conductive elements 2011, 2012 of the passive sensing means 201 -for instance the spires of the spiral inductor 101 and/or the various branches of the interdigitated capacitors 121, 122, 123, 124, 125, 126- form first electrodes for a plurality of sensing capacitors, and the areas opposite thereto on the surface 2061 form respective second electrodes of these sensing capacitors, without needing to physically build any second sensing electrodes in the passive sensing means 201. The deformation of the surface 2061 of the high relative permittivity layer 206 will affect the distance between these electrodes, thereby also affecting the resonance frequency. This variation can, in turn, be detected using an external magnetic field following known methods.

The same effect can be achieved between a conductive element on the backside 2022 of the carrier substrate 202 and the surface 2061, if this element is not screened by a conductive element 2011, 2012 of the front side 2021, like for instance the conductive element 2014 in Figure 2. This could be for instance the situation in the embodiments illustrated in Figures 5 to 7, wherein at least one spire 5012 or 6012 on the second main side of the carrier substrate has a larger diameter than any of the spires 5011 or 6012 on the first main side. In embodiment where a conductive element of the backside 2022 is, however, screened by a conductive element 2011 of the front side 2021, as also illustrated in Figure 2 with the conductive element 2013, a constant parasitic capacitance can exist between these two elements 2011, 2013, which would only be a fixed parameter and therefore not build a sensing capacitor. This could be, in turn, the case in the embodiment illustrated with reference to Figure 1, wherein the spire 1012 is screened by the outermost spire of the plurality of spires 1011.

By analogy, if a layer 208 of another high relative permittivity material is provided for instance over the carrier 205, as illustrated schematically in Figure 2, further parasitic capacitances could also exist between some of the conductive elements 2011, 2012, 2013, 2014 of the passive sensing means 201 and opposite areas of the layer 208, which could perturb the monitoring of the deformations of the surface 2061 of the first high relative permittivity layer 206. Thus, it is preferable that the carrier element 205 is manufactured in such a manner that the passive sensing means 201 can be attached thereto such that the distances D and D' from any of the coplanar conductive elements 2011, 2012 on the front side 2021 and from any of the coplanar conductive elements 2013, 2014 on the backside 2022 to the surface 2081 of the layer 208 are respectively greater than the distances d and d' from said coplanar conductive elements 2011, 2012 and 2013, 2014 to the opposite area of the surface 2061 of the layer 206. In this way, only the parasitic capacitances of the sensing capacitors can be main variable parameters as a function of the deformations of the surface 2061, while any other capacitance of the physiological parameter monitoring system 200 will be either fixed or negligible in comparison. Furthermore, it is also preferable that the thickness of the passive sensing means 201, in particular of the layer 202 of carrier substrate, or in other words the distance between the conductive element 2011, 2012 of the front side 2021 and the conductive elements 2013, 2014 on the backside 2022, is sufficiently small to consider that Do D' and d ≈ d'. Thus, in the following, reference can be made to D and d, without specifically distinguishing these values from D' and d', respectively.

In a preferred variant of the embodiment illustrated with reference to Figure 2, in particular in a variant wherein the passive sensing means 201 is the passive sensor 100 of the exemplary embodiment illustrated in Figure 1 or the passive sensors 500, 600, 700 of any of the variants illustrated in Figures 5 to 7, the first high relative permittivity layer 206 can be eye tissue such as the cornea and/or a tear film formed thereon, the second high relative permittivity layer 208 can be eye tissue such as the eyelid and/or a tear film formed between the eyelid and the carrier element 205, wherein the carrier element 205 can be a contact lens, and the low relative permittivity intermediate layer 207 can be an intermediate space filled with air or, in further variants, with a biocompatible low relative permittivity dielectric material. Finally, the physiological parameter can be the intraocular pressure, which can thus be monitored following the variations of the resonance frequency as a function of the distance variation between the passive sensor 100 and the surface of the eye, in particular the cornea and/or the tear film thereon. In that preferred variant, the inductor 101 and the plurality of capacitors 121, 122, 123, 124, 125, 126 can be chosen such that the initial resonance frequency of the passive sensor 100 is in the vicinity of 30 MHz. Near this frequency, the relative permittivity εᵣ for the different layers could then be: εᵣ (eyelid) ≈ 80 for the layer 208, and εᵣ (cornea) ≈ 100 and εᵣ (tear film) ≈ 80, such that it could be considered that εᵣ (cornea) ≈ εᵣ (tear film) near 30 MHz, for the layer 206. Furthermore, the relative permittivity of the material forming the contact lens 205, which could be silicon or a polymer material that can be used for rigid or soft contact lens elements, could be εᵣ (silicon) ≈ 3, and that of the dielectric material in the intermediate space 207, which could be air or another low relative permittivity biocompatible dielectric material, could be εᵣ (air, other dielectric) ≈ 1-3.

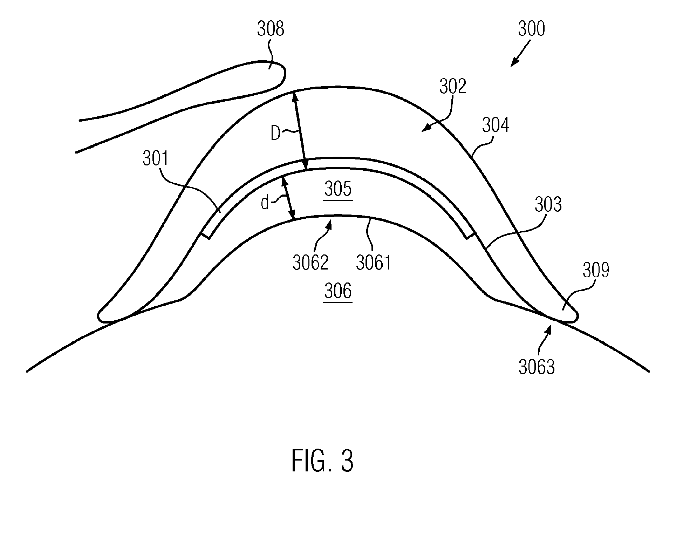

Figure 3 illustrates a preferred variant of the physiological parameter monitoring system 200 of the embodiment illustrated with reference to Figure 2, wherein variations of the intraocular pressure can be monitored. Thus, the embodiment illustrated in Figure 3 is in all aspects analog to the embodiment illustrated in Figure 2. In this variant, the physiological parameter monitoring system 300 can comprise a passive sensing means 301, which can be in particular the passive sensor 100 of the embodiment illustrated in Figure 1 or the passive sensors 500, 600, 700 of any of the variants illustrated in Figures 5 to 7, preferably attached at its backside to the inner surface 303 of a contact lens 302, such that the coplanar spires 1011 of the inductance 101 and the coplanar capacitors 121, 122, 123, 124, 125, 126 are arranged facing the surface 3061 of an eye 306, in particular of the cornea 3062, for which intraocular pressure variations will be monitored, while the backside is arranged for instance towards the eyelid 308. For simplicity purposes, the passive sensing means 301 is illustrated as a single layer, but the skilled person will understand that the configuration is analog to that of the embodiment illustrated in Figure 2 applied to the passive sensor 100 of Figure 1 or any of the passive sensors 500, 600, 700 of the embodiments illustrated with reference to Figures 5 to 7.

In the embodiment illustrated in Figure 3, the contact lens 302 also comprises an outer surface 304 adapted for contacting eye tissue and/or a tear film thereon. In particular, the inner surface 303 of the lens 302 is adapted for contacting at least the surface 3061 of the eye 306 and preferably also a tear film thereon, while the outer surface 304 of the lens 302 is adapted for contacting at least the eyelid 308 and preferably also a tear film. Furthermore, the contact lens 302 can preferably be a rigid contact lens of the scleral type, such that its peripheral area 309 rests on the surface 3061, in particular on the sclera 3063 and/or on the tear film formed thereon (the tear film is not illustrated for simplicity), and such that the lens 302 further provides an intermediate space 305, which can in particular be filled with air, between the surface 3061 of the eye 306 and the passive sensing means 301. Following a preferred variant of the embodiment illustrated in Figure 2, the thickness of the substrate layer 202, and therefore the distance between the spires 1011 on the first main side and the at least one spire 1012 on the second main side in the passive sensor 100, or by analogy in any of the passive sensors 500, 600, 700, can be less than about 100 µm, preferably even less than 50 µm, and the distance d ≈ d' between the passive sensing means 301 and the surface 3061 is smaller than the distance D ≈ D' between the passive sensing means 301 and the outer surface 304 of the contact lens 302, for instance, without limiting the present invention to these values, d ≈ d' ≈ 350 µm and D ≈ D' ≈ 500 µm, such that any parasitic capacitance between the passive sensing means 301 and the eyelid 308 will be either negligible or non-existent in comparison to the parasitic capacitances of the sensing capacitors formed between the passive sensing means 301 and the surface 3061.

The variant illustrated in Figure 3 can be particularly advantageous when the passive sensing means 301 is chosen with a low resonance frequency, for instance well below 30 MHz, at which the relative permittivity of the cornea becomes much larger than that of the tear film thereon, in other words at frequencies for which εᵣ (cornea) >> εᵣ (tear film). In that case, the tear film on the surface 3061 of the eye 306 can be considered to form part of the low permittivity intermediate space 305.

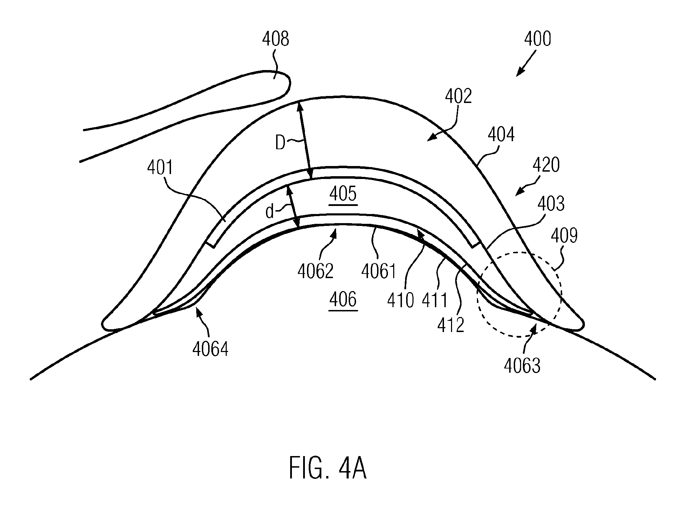

Figure 4A illustrates another preferred variant of a physiological parameter monitoring system 400, which is in most aspects similar to the physiological parameter monitoring system 300 of the embodiment illustrated in Figure 3. Thus, the physiological parameter monitoring system 400 is used for monitoring variations of the intraocular pressure in the eye 406, and comprises a passive sensing means 401, which can again preferably be the passive sensor 100 of the embodiment referring to Figure 1, or any of the variants described with reference to Figures 5 to 7, again preferably attached at its backside to the inner surface 403 of a first rigid contact lens or contact lens element 402, such that the coplanar conductive elements on the front side of the passive sensing means 401, for instance the spires 1011 of the inductance 101 and the coplanar capacitors 121, 122, 123, 124, 125, 126, are arranged facing the surface 4061 of the eye 406, in particular the cornea 4062.

In the embodiment illustrated in Figure 4A, the rigid contact lens element 402 also comprises an outer surface 404 adapted for contacting the eyelid 408 and tear film thereon, and its inner surface 403 is also preferably adapted for contacting at least the surface 4061 of the eye 406, preferably also the tear film thereon (the tear films are again not illustrated for simplicity), and in particular such that its peripheral area 409 rests on the sclera 4063 of the eye 406, providing also the intermediate space 405.

However, in the variant illustrated in Figure 4A, the passive sensing means 401 is chosen with a resonance frequency around 30 MHz, such that εᵣ (cornea) ≈ εᵣ (tear film) >> εᵣ (air) ≈ εᵣ (lens material). Thus, in order to efficiently detect any deformation of the surface 4061, or in other words in order to provide for at least one sensing capacitor between the coplanar elements of the passive sensing means 401 and opposite areas of the surface 4061, it is necessary to avoid that the space 405 is filled with tear film. Thus, in the variant illustrated in Figure 4A, the physiological parameter monitoring system 400 comprises a multilayered contact lens 420, comprising the rigid lens 402, as well as a soft layer 410, which can be for instance a soft contact lens, joined at the edges towards the peripheral area 409, enclosing the intermediate space 405. The soft lens 410 also comprises an inner surface 411 adapted for contacting the surface 4061 and tear film thereon, as well as an outer surface 412 opposite the inner surface 411. In order to improve the contact with the surface 4061 of the eye 406, in particular of the cornea 4062, the multilayered lens 420, and in particular the soft lens 410, can avoid contact around the limbus area 4064 of the eye 406, while the peripheral area 409 rests on the sclera 4063.

Thus, in the variant illustrated in Figure 4A, the tear film on the surface 4061 of the eye 406 can be considered to form part of the cornea 4062, while the soft lens 410 can be considered as one with the intermediate space 405, which can be filled with air or any other compressible and biocompatible material with a comparable low relative permittivity. In this embodiment, the distance d ≈ d' from the passive sensing means 401 to the inner surface 411 of the soft lens 410, in other words to the interface between the soft lens 410 and the tear film on the corneal area 4062, is smaller than the distance D = D' between the passive sensing means 401 and the outer surface 404 of the rigid contact lens 402, such that any parasitic capacitance between the passive sensing means 401 and the eyelid 408 will be either negligible or non-existent compared to the parasitic capacitances of the sensing capacitors formed by the passive sensing means 401 and respective opposite areas of the surface 4061.

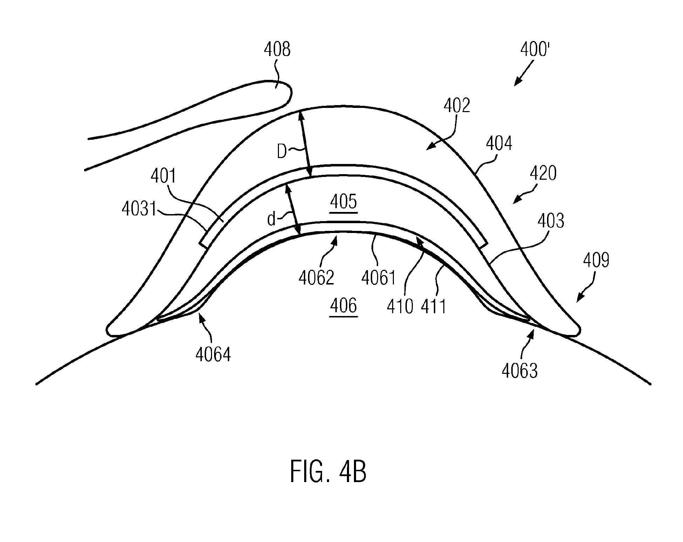

Figure 4B illustrates a variant of a physiological parameter monitoring system 400', which is essentially the same as the physiological parameter monitoring system 400 illustrated in Figure 4A, with the exception that the sensing means 401 is accommodated in a recess 4031 provided in the inner surface 403 of the rigid part 402 of the multilayered lens 420. This variant is then more advantageous than the previous variant in terms of attachment stability of the passive sensing means 401 within the multilayered lens 420.

Figures 5 to 7 illustrate further embodiments of passive sensing means according to the present invention. As mentioned above, these variants can all be used as alternatives to the variant illustrated in Figure 1, and in particular these variants can all be used in the physiological monitoring systems 200, 300, 400, 400' described in the embodiments referring to Figures 2 to 4. The reader is therefore referred back to the description above regarding any features of the passive sensors 500, 600, 700 of the embodiments illustrated in Figures 5 to 7 that are analog to those of the passive sensor 100 illustrated in Figure 1, as well as regarding the use in combination with any of the physiological parameter monitoring systems 200, 300, 400, 400' of the embodiments illustrated in Figures 2 to 4.

In the embodiment illustrated in Figure 5, like the passive sensor 100 of the embodiment illustrated in Figure 1, the passive sensor 500 is a resonant circuit comprising an inductive element, here inductor 501, having first spires 5011 on a first main side of a carrier substrate and at least one spire 5012 on a second side of the carrier substrate, opposite the first main side. However, in contrast with the passive sensor 100 illustrated in Figure 1, while the spires 5011 on the first main side have the same configuration as the spires 1011 of the inductor 101, in the embodiment illustrated in Figure 5, the outermost spire of the at least one spire 5012 on the second main side is on a larger circumference than any of the spires 5011 of the first main side. Thus, while Figure 5 schematically illustrates only one spire 5012, it would be possible to have more than one spire 5012 on the second main side before the inductor 501 continues on the first main side with the via 5053.

Other than the difference relating to the at least one spire 5012 on the second main side, the inductor 501 of the embodiment illustrated in Figure 5 is substantially of the same type as the inductor 101 of the embodiment illustrated in Figure 1. Thus, it can also comprise concave arc-shaped segments 5071, 5072, 5073 with respect to -but not centered on- a substantially central reference point 510 of the passive sensor 500, as well as convex arc-shaped segments 5021, 5022, 5023 joining the concave segments 5071, 5072, 5073 to one another. Similarly, the inductor 501 can further comprise straight inductor segments 5031, 5032, 5033, 5034, 5035, 5036 and rounded junctions 5041, 5042, 5043, 5044, 5045, 5046 between the straight segments 5031, 5032, 5033, 5034, 5035, 5036 and the concave segments 5071, 5072, 5073.

Also like the inductor 101 illustrated in Figure 1, the inductor 501 of the embodiment illustrated in Figure 5 can also comprise a first terminal 5051 on the innermost spire of the plurality of spires 5011 on the first main side of the carrier substrate, and a second terminal 5052 on the outermost circumference of the at least one spire 5012 on the second main side of the carrier substrate, as well as small deflected areas 506 between successive spires. The first main side of the inductor 501 can also comprise for instance between about 5 to 20 spires 5011, preferably 8 to 15 spires 5011, more preferably 10 to 13 spires 5011, and its width can also preferably be kept below about 2.0 mm, for instance at about 1.5 mm or even below. Also similarly, the second main side can comprise up to 5 spires 5012. However, for the sake of simplicity, the inductor 501 is illustrated in Figure 5 with the same amount of spires 5011, 5012 on the first and second main sides as the inductor 101 in Figure 1.

In the embodiment illustrated in Figure 5, the passive sensor 500 further comprises at least one capacitive element, here the three capacitors 521, 522, 523, which are all coplanar in one layer with the spires 5011 on the first main side of the carrier substrate, prior to any deformation of the passive sensor 500 for its incorporation in a contact lens of a physiological parameter monitoring system. In contrast with the embodiment illustrated in Figure 1, only one capacitor 521, 522, 523 is provided at the inner circumference of each concave arc-shaped segment 5071, 5072, 5073, respectively. Like in the embodiment illustrated in Figure 1, each capacitor 521, 522, 523 is also coplanar, but the first and second electrodes in each pair of electrodes 5211 and 5212, 5221 and 5222, 5231 and 5232, are not interdigitated with each other. However, as illustrated in Figure 5, each individual electrode 5211, 5212, 5221, 5222, 5231, 5232 is itself an interdigitated electrode. As further illustrated, each individual electrode 5211, 5212, 5221, 5222, 5231, 5232 of the passive sensor 500 can cover roughly at least as much surface as a full interdigitated capacitor 121, 122, 123, 124, 125, 126 of the passive sensor 100 of the embodiment referring to Figure 1. In terms of shape, compared to the embodiment illustrated in Figure 1, in the embodiment illustrated in Figure 5, each electrode 5211, 5212, 5221, 5222, 5231, 5232 roughly corresponds to having the two interdigitated E-shaped electrodes 1211 and 1212, 1221 and 1222, 1231 and 1232, 1241 and 1242, 1251 and 1252, and 1261 and 1262 of each capacitor 121, 122, 123, 124, 125, 126 joined at their largest extremity -towards the innermost spire of the inductor 101- thereby forming a single integral interdigitated electrode. An advantage of shaping the individual electrodes 5211, 5212, 5221, 5222, 5231, 5232 in this way is that it facilitates the molding or shaping of the passive sensor 500 for its attachment to a contact lens. As illustrated in Figure 5, in an analog manner to the embodiment illustrated in Figure 1, the back of the electrodes 5211, 5212, 5221, 5222, 5231, 5232 facing the convex arc-shaped segments 5021, 5022, 5023 of the inductor 501 can also follow the arc-shaped geometry of the convex arc-shaped segments 5021, 5022, 5023 and broaden towards the central area 530, with the same advantage over the prior art of improving the coverage of the underlying surface of the eye, in particular over the cornea, once the passive sensor 500 is integrated in a physiological parameter monitoring system, for instance in any of the physiological parameter monitoring systems 200, 300, 400, 400'.

Furthermore, like in the embodiment illustrated in Figure 1, the first electrodes 5211, 5221, 5231 of the passive sensor 500 can be provided as integral extensions of the innermost of the spires 5011 on the first main side of the carrier substrate, while the second electrodes 5212, 5222, 5232 can be connected to the spire 5012 of the second main side by means of respective electrically conductive vias 5213, 5223, 5233, which can also comprise a respective bridge with the spire 5012. Figure 5 also illustrates that the vias 5213, 5223, 5233 can cross the substrate and even the second electrodes 5212, 5222, 5232. An advantage of this configuration in the embodiment illustrated in Figure 5 is, in comparison to the embodiment illustrated in Figure 1, that the number of electrically connecting vias is halved, thereby reducing the amount of areas where material crosses the carrier substrate, while keeping at least the same amount of surface covered by coplanar capacitors.

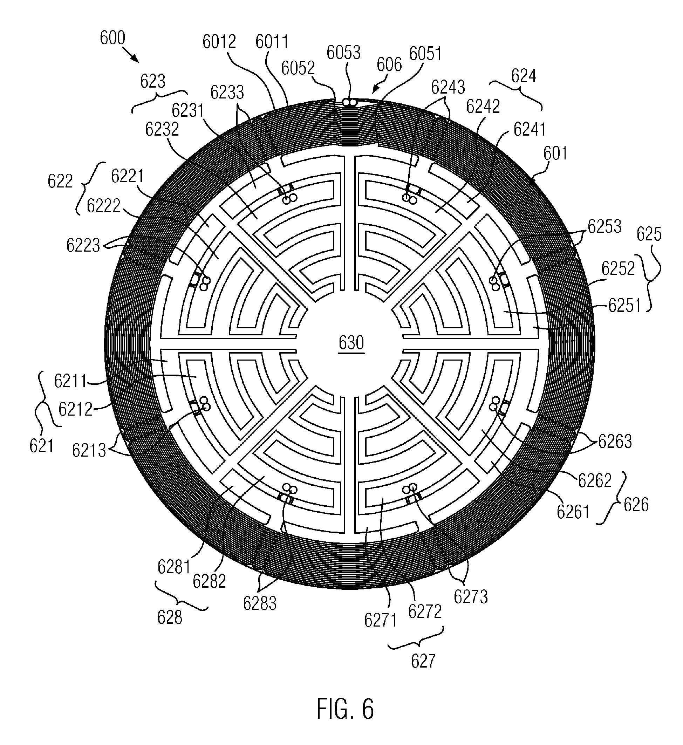

In the embodiment illustrated in Figure 6, like the passive sensors 100, 500 of the embodiments illustrated in Figures 1 and 5, the passive sensor 600 is also a resonant circuit comprising an inductive element, here inductor 601, with more spires 6011 on the first main side of a carrier substrate than spires 6012 on the second main side thereof. The passive sensor 600 also comprises at least one capacitive element, here the plurality of coplanar capacitors 621, 622, 623, 624, 625, 626, 627, 628, which are in particular also coplanar in one layer with the spires 6011 on the first main side prior to any deformation of the passive sensor 600 for its incorporation in a contact lens of a physiological parameter monitoring system. Again, for clarity purposes, the carrier substrate and any optional protective coating layers thereon are not illustrated.

Following a preferred variant, in alternative to the embodiments illustrated in Figures 1 and 5, the inductor 601 of the embodiment illustrated in Figure 6 is a circular ring-shaped inductor spiraling from a first terminal 6051 on the innermost spire of the plurality of spires 6011 on the first main side towards a second terminal 6052 on the outermost circumference of the at least one spire 6012 on the second main side, and the connection between the spires 6011, 6012 can be provided through the carrier substrate by means of a conductive via 6053. While the inductors 101 and 501 of the previous embodiments and their variants can be more advantageous in terms of facilitating the deformation of the passive sensors 100, 500 in view of their attachment to the concave cap-shape of a contact lens, the ring-shaped inductor 601 of the passive sensor 600 of the embodiment illustrated in Figure 6 is in turn more advantageous in terms of the amplitude of the signal at the antenna of a complementary portable device generating the external magnetic field. Like in the previous embodiments, the inductor 601 can also comprise for instance about 5 to 20 spires 6011 on the first main side, preferably 8 to 15 spires 6011, more preferably 10 to 13 spires 6011, and up to 5 spires 6012 on the second main side. Its width can also preferably be kept below about 2.0 mm, for instance at about 1.5 mm or even below. Like the inductors 101, 501 of the embodiments illustrated in Figures 1 and 5, the inductor 601 of the embodiment illustrated in Figure 6 is shown with 12 spires 6011 on the first main side, which can have a width of about 50 µm and be spaced apart by also about 50 µm, and one spire 6012 on the second main side. However, also like in the previous embodiments, the width of the spires 6011, 6012 and/or the distance between successive spires 6011 or 6012 could be chosen in a range from about 30 µm to about 100 µm, preferably between about 40 µm and about 80 µm and don't need to be the same.

In order to provide sufficient surface coverage in view of using the passive sensor 600 for detecting deformations of the surface of an eye while still providing for sufficient flexibility for an attachment to a contact lens, in the embodiment illustrated in Figure 6, a plurality of capacitors are provided, here the eight coplanar interdigitated capacitors 621, 622, 623, 624, 625, 626, 627, 628. In view of the description above, the skilled person will understand that this number should not be seen as restrictive, and that more or less capacitors can be used depending on the desired configuration and sensitivity of the passive sensing means, as well as on the underlying surface coverage.

As further illustrated in Figure 6, the capacitors 621, 622, 623, 624, 625, 626, 627, 628 are very similar to the capacitors of the embodiments illustrated in Figures 1 and 5. Thus, the pairs of electrodes 6211 and 6212, 6221 and 6222, 6231 and 6232, 6241 and 6242, 6251 and 6252, 6261 and 6262, 6271 and 6272, and 6281 and 6282 are also coplanar to one another, forming interdigitated E-shapes. Furthermore, the capacitors 621, 622, 623, 624, 625, 626, 627, 628 can also be larger outwards from the central area 630 than towards the central area 630 but, in contrast to Figures 1 and 5, do not broaden again towards the central area 630, such that the overall shape of each capacitor 621, 622, 623, 624, 625, 626, 627, 628 is essentially trapezoidal but also arched following the shape of the inductor 101, with the larger base facing outwards from the central area 630 and the smaller base facing towards said central area 630, with the same advantages as described for the previous embodiments.

Furthermore, as described also for the embodiments illustrated in Figures 1 and 5, in the passive sensor 600 of the embodiment illustrated in Figure 6, the first electrodes 6211, 6221, 6231, 6241, 6251, 6261, 6271, 6281 can also be electrically connected to and be provided as integral extensions of the innermost spire of the plurality of spires 6011 on the first main side of the inductor 601, while the second electrodes 6212, 6222, 6232, 6242, 6252, 6262, 6272, 6282 can be connected to the spire 6012 on the second main side by means of respective electrically conductive vias 6213, 6223, 6233, 6243, 6253, 6263, 6273, 6283 as also described above for the previous embodiments.

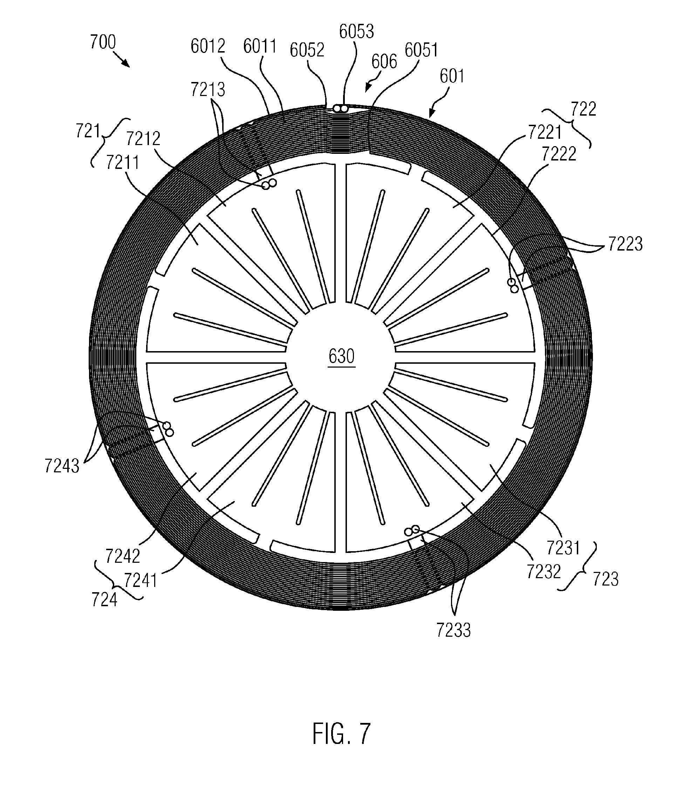

In the embodiment illustrated in Figure 7, the passive sensor 700 is also a resonant circuit comprising an inductive element, here the same inductor 601 as in the embodiment illustrated in Figure 6, and at least one capacitive element, here the four capacitors 721, 722, 723, 724, which are all coplanar in one layer prior to any deformation of the passive sensor 700 for its incorporation in a contact lens of a physiological parameter monitoring system. The reader is referred to the description above in particular regarding specifically the inductor 601, as well as other features in common with the passive sensors 100, 500, 600 of the previous embodiments.

In contrast with the embodiment illustrated in Figure 6, but similarly to the embodiment illustrated in Figure 5, in the passive sensor 700, the first and second electrodes in each pair of electrodes 7211 and 7212, 7221 and 7222, 7231 and 7232, 7241 and 7242 of the capacitors 721, 722, 723, 724 are not interdigitated with each other. However, as illustrated in Figure 7, each individual electrode 7211, 7212, 7221, 7222, 7231, 7232, 7241, 7242 can be in the shape of a trident pointing towards the central area 630, forming also an overall trapezoidal shape as described above, wherein the capacitors 721, 722, 723, 724 are also larger towards the outside than towards the central area 630 and their larger base can follow the rounded geometry of the inductor 601, with again the same advantages as described above for instance for the embodiment illustrated in Figure 1. As further illustrated in Figure 7, each individual electrode 7211, 7212, 7221, 7222, 7231, 7232, 7241, 7242 of the passive sensor 700 can cover roughly at least as much surface as a full interdigitated capacitor 621, 622, 623, 624, 625, 626, 627, 628 of the embodiment illustrated in Figure 6.

Furthermore, like in the previous embodiments, the first electrodes 7211, 7221, 7231, 7241 of the passive sensor 700 can be electrically connected to the innermost spire of the plurality of spires 6011 on the first main side of the inductor 601 and can be provided as integral extensions thereof, while the second electrodes 7212, 7222, 7232, 7242 can be connected to the spire 6012 on the second main side by means of respective electrically conductive vias 7213, 7223, 7233, 7243 and respectively associated conductive bridges. Like in the embodiment illustrated in Figure 5, an advantage of this configuration in the embodiment illustrated in Figure 7 is, in comparison to the embodiment illustrated in Figure 6, that the number of electrically connecting vias is halved, thereby reducing the amount of areas where material crosses the carrier substrate, while keeping at least the same amount of surface covered by coplanar capacitors.

As mentioned above, any of the passive sensors 100, 500, 600, 700 of the embodiments illustrated in Figures 1 or 5 to 7, or any of their variants, can be used in the physiological parameter monitoring systems 200, 300, 400, 400' of the embodiments illustrated in Figures 2 to 4B or variants thereof. Indeed, when the passive sensing means 100, 500, 600, 700 respond to an external magnetic field generated by a complementary portable device, the configuration of the inductor and/or of the coplanar capacitors in each of the passive sensors 100, 500, 600, 700 allows the electric field lines generated therein to protrude out of the plane of the passive sensor 100, 500, 600, 700. Thus, when used in any of the physiological parameter monitoring systems 200, 300, 400, 400', parasitic capacitances will exist with the underlying eye tissue and/or tear film thereon, which will form sensing capacitors, wherein the spires of the inductor and/or the physical capacitors of the passive sensors 100, 500, 600, 700 are first electrodes of said sensing capacitors, and the eye tissue and/or the tear film are the second electrodes thereof.

The skilled person will find it obvious that the embodiments described above can be combined in order to provide further embodiments of the various aspects of the present invention. In particular, the variants of a passive sensing means can all be used in either variant of the physiological parameter monitoring systems.

The skilled person will also appreciate that the present invention provides an improvement in the field of passive sensing devices for monitoring variations of a physiological parameter, in particular for monitoring variations of the intraocular pressure. The passive sensing device and the physiological parameter monitoring device according to preferred variants of aspects of the present invention can be used advantageously for patients suffering from glaucoma and related eye diseases. Compared to solutions known in the art, the aspects of the present invention provide a sensing device with improved sensitivity and improved flexibility for integration or attachment in a contact lens. The invention also provides a new physiological parameter monitoring system for detecting variations of parameters that can be correlated to variations of the physiological parameter.

## Claims

1. Passive sensing means (100) for a contact lens of a physiological parameter monitoring system, for detecting variations of a physiological parameter, in particular intraocular pressure, the passive sensing means (100) forming a resonant circuit comprising:
an inductor (101) comprising spires (1011) on a first main side of a carrier substrate and at least one spire (1012) on a second main side of said carrier substrate, opposite the first main side; and
at least one capacitor (121, 122, 123, 124, 125, 126),
**characterized in that**
said at least one capacitor (121, 122, 123, 124, 125, 126) is coplanar.

2. Passive sensing means (100) for a contact lens of a physiological parameter monitoring system, for detecting variations of a physiological parameter, in particular intraocular pressure, the passive sensing means (100) forming a resonant circuit comprising:
an inductor (101) comprising spires (1011) on a first main side of a carrier substrate and at least one spire (1012) on a second main side of said carrier substrate, opposite the first main side; and
at least one capacitor (121, 122, 123, 124, 125, 126),
**characterized in that**
the passive sensing means (100), in particular the inductor (101) and/or said at least one capacitor (121, 122, 123, 124, 125, 126), forms first electrodes of at least one sensing capacitor.

3. Passive sensing means (100) according to any of claims 1 or 2, wherein said at least one capacitor (121, 122, 123, 124, 125, 126) comprises a first electrode (1211, 1221, 1231, 1241, 1251, 1261) and a second electrode (1212, 1222, 1232, 1242, 1252, 1262), and wherein said first and second electrodes are coplanar with the spires (1011) of the inductor (101) on the first main side, in particular only on the first main side.

4. Passive sensing means (100) according to claim 3, wherein said first electrode (1211, 1221, 1231, 1241, 1251, 1261) is electrically connected to an inner circumference of the spires (1011) on the first main side and said second electrode (1212, 1222, 1232, 1242, 1252, 1262) is electrically connected, in particular by means of an electrically conductive via (1213, 1223, 1233, 1243, 1253, 1263), to said at least one spire (1012) on the second main side.

5. Passive sensing means (100) according to any of claims 3 or 4, wherein said at least one capacitor (121, 122, 123, 124, 125, 126) and/or said first electrode (1211, 1221, 1231, 1241, 1251, 1261) and second electrode (1212, 1222, 1232, 1242, 1252, 1262) is/are interdigitated.

6. Passive sensing means (100) according to any of the preceding claims, wherein said at least one capacitor (121, 122, 123, 124, 125, 126) is provided at an inner circumference of the inductor (101), in particular of the spires (1011) on the first main side, more in particular towards a central area (130) of said passive sensing means (100).

7. Passive sensing means (100) according to claim 6, wherein said at least one capacitor (121, 122, 123, 124, 125, 126) is larger towards the inner circumference of the inductor (101) than towards said central area (130).

8. Passive sensing means (100) according to any of claims 6 or 7, wherein said at least one capacitor (121, 122, 123, 124, 125, 126) is partially arc-shaped following said inner circumference.

9. Passive sensing means (100) according to any of the preceding claims, wherein the inductor (101) comprises less spires on the second main side than on the first main side of the carrier substrate.

10. Passive sensing means (100) according to any of the preceding claims, wherein said at least one spire (1012) on the second main side is only provided at an outer circumference.

11. Passive sensing means (600) according to any of the preceding claims, wherein the inductor (601) is circular ring-shaped.

12. Passive sensing means (100) according to any of claims 1 to 10, wherein the inductor (101) is a flat spiral inductor comprising a plurality of, preferably three, concave arc-shaped segments (1071, 1072, 1073) with respect to a substantially central point (110) of said passive sensing means (100), and wherein for at least one, preferably all, of the plurality of concave arc-shaped segments (1071, 1072, 1073), the radius of curvature of said at least one segment at a point thereof is greater than the distance between said point and said substantially central point (110).

13. Passive sensing means (100) according to claim 12, wherein the inductor (101) further comprises convex arc-shaped segments (1021, 1022, 1023) arranged between the concave arc-shaped segments (1071, 1072, 1073).

14. Passive sensing means (100) according to claim 13, wherein the inductor (101) further comprises straight segments (1031, 1032, 1033, 1034, 1035, 1036) joining said convex arc-shaped segments (1021, 1022, 1023) to said concave arc-shaped segments (1071, 1072, 1073), and wherein the junctions (1041, 1042, 1043, 1044, 1045, 1046) between said straight segments (1031, 1032, 1033, 1034, 1035, 1036) and the concave arc-shaped segments (1071, 1072, 1073) are preferably rounded.

15. Passive sensing means (100) according to any of the preceding claims, wherein the inductor (101) comprises 5 to 20 spires (1011), in particular 8 to 15 spires (1011), more in particular 10 to 13 spires (1011), on the first main side and one or more spires (1012), in particular up to 5 spires (1012), on the second main side of the carrier substrate.

16. Physiological parameter monitoring system (200, 300, 400, 420), in particular for detecting variations of intraocular pressure, comprising:
a first contact lens element (302, 402), preferably of a rigid polymer material, having an inner surface (303, 403) and an outer surface (304, 404) opposite the inner surface (303, 403), being adapted for contacting an ocular tissue (306, 308; 406, 408), preferably with a peripheral area (309, 409) adapted for contacting the sclera (3063, 4063) such that an intermediate space (305, 405) is provided between the inner surface (303, 403) and an eye surface (3061, 4061) when the peripheral area (309, 409) contacts the sclera (3063, 4063); and
further comprising a passive sensing means (100, 201, 301, 401, 500, 600, 700) according to any of claims 1 to 15.

17. Physiological parameter monitoring system (200, 300, 400, 420) according to claim 16, wherein the passive sensing means (201, 301, 401) is provided at the inner surface (303, 403) of said first contact lens element (205, 302, 402), in particular in a recess (4031) of said inner surface (403), preferably with the second main side of the carrier substrate towards said inner surface (303, 403).

18. Physiological parameter monitoring system (400, 420) according to any of claims 16 or 17, further comprising a second contact lens element (410), preferably of a flexible material, in particular a flexible polymer material, more in particular a hydrophilic flexible polymer material, having an inner surface (411) and an outer surface (412) opposite the inner surface, wherein at least the inner surface is adapted for contacting an ocular tissue (406), in particular at least the cornea (4061, 4062) and/or a tear film thereon, and wherein the first lens element (402) and the second lens element (410) are attached to one another at a peripheral attachment area (409), thereby enclosing an intermediate space (405).
